# EUROPEAN PATENT APPLICATION

(11) **EP 1 733 691 A1**
(43) Date of publication of application: **20.12.2006**
(21) Application number: 05105195.1
(22) Date of filing: 14.06.2005
(51) Int. Cl.: A61B 18/20

(54) **Apparatus for cosmetic skin rejuvenation treatment**

(71) Applicant: Koninklijke Philips Electronics N.V., 5621 BA Eindhoven (NL)
(72) Inventor: The designation of the inventor has not yet been filed
(74) Representative: Pet, Robert Jacob

(57) **Abstract**

The present invention relates to an apparatus for cosmetic skin rejuvenation treatment comprising a continuous wave light source having a high intensity discharge lamp (104; 204; 304; 604), the high intensity discharge lamp having at least one predominant spectral peak between 600 and 700nm.

## Description

### FIELD OF THE INVENTION

The present invention relates to the field of phototherapy of the skin.

### BACKGROUND AND PRIOR ART

Various apparatuses and methods for light therapy are known from the prior art. For example, US5,549,660 shows a method of treating acne. The acne treatment is performed by illumination using light emitting diodes in a continuous wave mode of operation to generate continuous wave light radiation in a narrow bandwidth. The continuous wave light radiation is concentrated and projected onto an acne-affected dermal zone for biostimulative treatment.

In addition, high intensity visible light phototherapy for acne was described by Meffert et al, (Dermatol-Monatsschr. 1990; 176(10): 597-603), and in US Patents 6,676,655 and US 6,626,932.

### SUMMARY OF THE INVENTION

In accordance with the present invention there is provided an apparatus for cosmetic skin rejuvenation treatment comprising a continuous wave light source having a high intensity discharge lamp, the high intensity discharge lamp having at least one predominant spectral peak between 550 and 700nm, preferably between 650 to 700 nm.

It has been found that treatment of the human skin with radiation of a wavelength between 550 to 700nm can reduce wrinkles. Usage of a high intensity discharge lamp for generation of this radiation is cost efficient. The present invention thus facilitates the design of a relatively inexpensive consumer device for skin rejuvenation treatment.

In accordance with an embodiment of the invention the high intensity discharge lamp has a salt filling. When the high intensity discharge lamp is switched on, the salt evaporates. The evaporated salt shifts the wavelength of the radiation emitted by the high intensity discharge lamp into the 550 to 700nm window that is required for the skin rejuvenation treatment.

In accordance with an embodiment of the invention the salt filling comprises a halide, such as lithium halide, sodium halide and/or calcium halide, in particular lithium iodide (LiI), sodium iodide (NaI) and/or calcium iodide (CaI₂) and/or respective bromides, fluorides, and/or chlorides. In addition the salt can contain other metal halides to stabilize the arc of the discharge lamp.

Preferably a lithium halide lamp having a spectral peak between 550 to 700nm, preferably between 650 to 700 nm, is used for wrinkle reduction. For example, the treatment is performed with a fluence of>10 J/cm² and a treatment time of above 100 seconds.

In accordance with an embodiment of the invention the high intensity discharge lamp has a gas filling containing argon, xenon, krypton, and/or neon.

The high intensity discharge lamp may or may not have a mercury filling. In accordance with an embodiment of the invention the plasma chamber of the high intensity discharge lamp has a quartz glass wall. The quartz glass has a silicon dioxide (SiO2) content of at least 95% by weight.

In accordance with an embodiment of the invention the plasma chamber of the high intensity discharge lamp has a ceramic wall, such as a PCA (Poly Cristalline Alumina; AL2O3 in polycrystalline form) wall.

In accordance with an embodiment of the invention the continuous wave light source can operate with an alternative high intensity discharge lamp for treatment of a skin disorder, such as for acne treatment. The alternative high intensity discharge lamp has at least one spectral peak between 390 and 430nm. Such a radiation spectrum is obtained e.g. by means of a halide salt filling, such as gallium iodide (GaI₃) and/or indium iodide (InI3) salt filling. Again, the gas filling can be argon, xenon, krypton and/or neon with or without mercury.

In accordance with an embodiment of the invention the continuous wave light source has means for (releasably) receiving the high intensity discharge lamp or the alternative high intensity discharge lamp. For example, for wrinkle treatment the high intensity discharge lamp having a spectral peak between 550 to 700nm is inserted into the light source, whereas for treatment of a skin disorder, such as for acne treatment, the high intensity discharge lamp is replaced with the alternative high intensity discharge lamp that has at least one predominant peak in the 390 to 430nm region.

In accordance with an embodiment of the invention the high intensity discharge lamp and the alternative high intensity discharge lamp are mounted on a rotational axis for pivoting one or the other lamp into a use position.

In accordance with still another embodiment of the present invention both the high intensity discharge lamp and the alternative high intensity discharge lamp are permanently mounted within the light source, such as close to the focal point of a reflector. The apparatus has a control element, such as a control button, for a user's selection of a first or a second mode of operation.

In the first mode of operation at least the high intensity discharge lamp with the peak in the 550 to 700nm region is switched on for skin rejuvenation treatment whereas in the second mode of operation at least the alternative high intensity discharge lamp is switched on for treatment of a skin disorder, such as acne.

Rather than switching the lamps on or off depending on the mode it is also possible to vary the respective intensities of the radiation. For example, in the first mode of operation the high intensity discharge lamp having the peak in the 550 to 700nm region is operated to produce a high intensity radiation whereas the alternative high intensity discharge lamp is operated at a low radiation intensity level. In the second mode of operation the alternative high intensity discharge lamp having a spectral peak in the 390 to 430nm region is operated with a high radiation intensity whereas the high intensity discharge lamp that has the spectral peak in the 550 to 700nm region is operated with a lower radiation intensity level.

In accordance with an embodiment of the invention both skin rejuvenation treatment, such as anti-wrinkle treatment, and treatment of a skin disorder, such as acne treatment, can be accomplished with a single lamp that has a filling comprising at least two different salts, such as lithium iodide and gallium iodide, resulting in respective spectral peaks.

In accordance with an embodiment of the invention the apparatus for skin rejuvenation treatment is portable. Preferably, the apparatus is a table-top unit. This is particularly advantageous for consumer devices as a need for an expensive mechanical support, such as a tripod, for holding and positioning the apparatus can be avoided.

### BRIEF DESCRIPTION OF THE DRAWINGS

In the following preferred embodiments of the present invention are described in greater detail by making reference to the drawings in which:
Figure 1 is a schematic cross-sectional view of a first embodiment of an apparatus of the invention,
Figure 2 is a schematic cross-sectional view of a second embodiment of an apparatus of the invention,
Figure 3 shows the embodiment of fig. 2 after pivoting of the lamps,
Figure 4 is a schematic cross-sectional view of a third embodiment of an apparatus of the invention,
Figure 5 is a cross-sectional view of a quartz high intensity discharge lamp,
Figure 6 is a cross-sectional view of a PCA high intensity discharge lamp,
Figure 7 a spectrum of a high intensity discharge lamp with a lithium iodide (LiI) salt filling,
Figure 8 a spectrum of a high intensity discharge lamp with a gallium iodide (GaI) salt filling,
Figure 9 a spectrum of a high intensity discharge lamp with both a lithium iodide (LiI) and a gallium iodide (GaI3) salt filling,
Figure 10 a table-top embodiment of an apparatus of the invention in a use position,
Figure 11 an embodiment of an apparatus of the invention to be hung to a wall in a use position.

### DETAILED DESCRIPTION

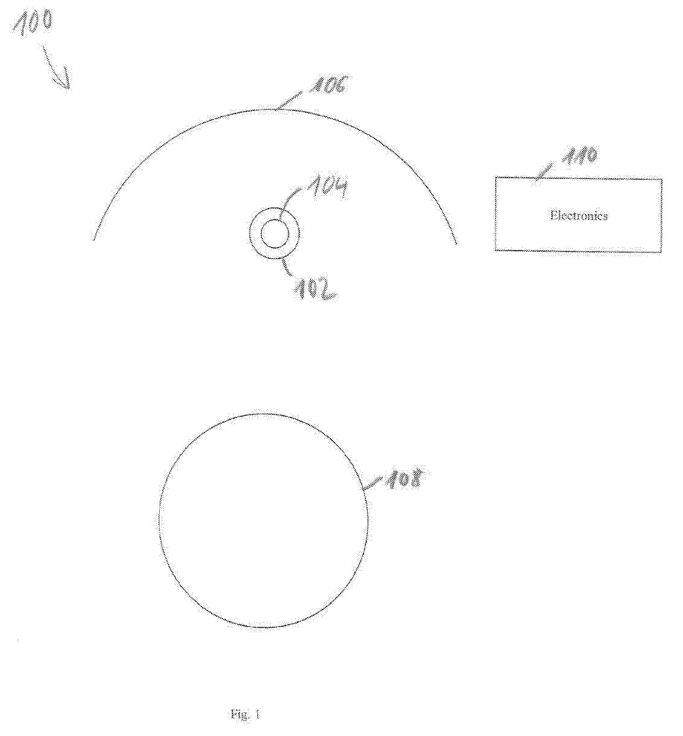

Fig. 1 shows an apparatus 100 for cosmetic skin rejuvenation treatment. The apparatus has a continuous wave light source that comprises a socket 102 for receiving high intensity discharge lamp 104. The high intensity discharge lamp 104 is positioned in a focal area of a reflector 106. The reflector 106 concentrates and directs the light emitted from the high intensity discharge lamp 104 towards a person 108 that is sitting in front of the apparatus 100. The operation of the apparatus 100 is controlled by an electronics module 110 that also has a current limiting function for operation of the high intensity discharge lamp 104. This current limiting function can be implemented as an inductor, a transformer ballast or, alternatively, using an electronic lamp driver module operating with a square wave current profile or a high frequency sinusoidal current profile

The high intensity discharge lamp 104 has a predominant spectral peak between 550 to 700 nm.

Preferably the skin of the person 108 is exposed to a light dose of at least 10J/cm² or above. For example, the high intensity discharge lamp 104 has a radiation efficiency above 16 % and a power consumption above 200 W. Such a set-up provides an efficient skin rejuvenation treatment, such as wrinkle treatment, for the person 108.

The high intensity discharge lamp 104 can be removed from the socket 102 and an alternative high intensity discharge lamp having another radiation spectrum can be plugged into the socket for another application.

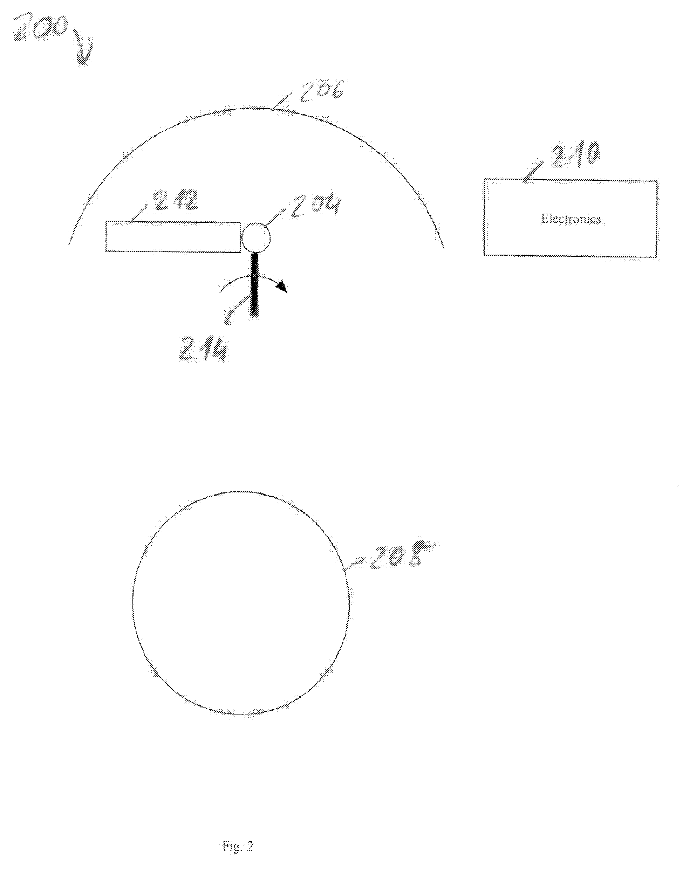

Fig. 2 shows an alternative embodiment. In the following figures like reference numerals will be used to designate like elements.

The apparatus 200 is a dual mode device that can be used both for skin rejuvenation, such as wrinkle treatment, and treatment of skin disorders, such as acne treatment. For this purpose, the apparatus 200 has an additional high intensity discharge lamp 212 that is mounted together with the high intensity discharge lamp 204 on a rotational axis 214 of the apparatus 200. The high intensity discharge lamp 204 has at least one predominant peak in the 550 to 700nm region and is used for skin rejuvenation treatment, whereas the high intensity discharge lamp 212 has at least one predominant peak in the 390 to 430nm region for treatment of skin disorders, such as acne treatment.

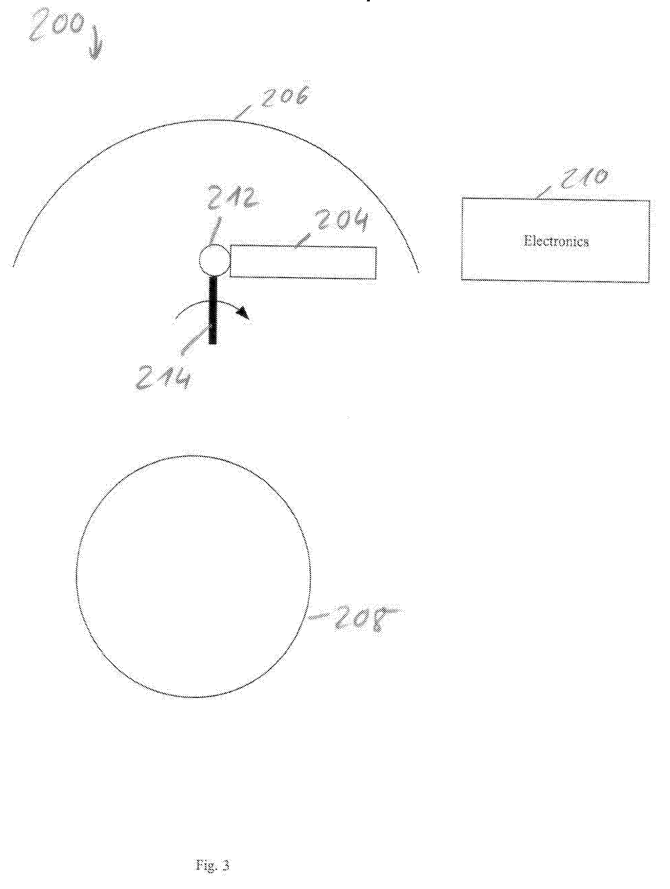

As shown in fig. 2, the high intensity discharge lamp 204 and the high intensity discharge lamp 212 are arranged perpendicularly. When the high intensity discharge lamp 204 is in a vertical position, as shown in fig. 2, the apparatus 200 can be operated in a first mode for skin rejuvenation. By pivoting the high intensity discharge lamp 204 and the high intensity discharge lamp 212 around the rotational axis 214 the high intensity discharge lamp 204 is brought into a horizontal position and the high intensity discharge lamp 212 is brought into a vertical position as shown in fig. 3. After pivoting the high intensity discharge lamp 212 in its vertical position, the apparatus 200 can be used in its second mode of operation for treatment of a skin disorder. The electronics module 210 serves for powering either the high intensity discharge lamp 204 or the high intensity discharge lamp 212 depending on the selected mode of operation of the apparatus 200.

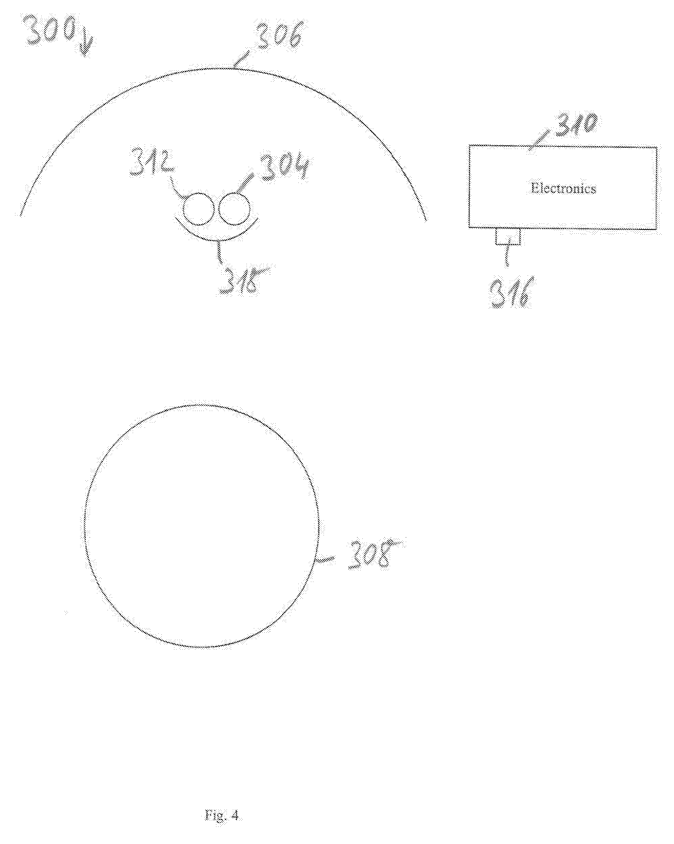

Fig. 4 shows still another embodiment of an apparatus 300. The apparatus 300 has both a high intensity discharge lamp 304 having at least one predominant peak in the 550 to 700nm range and a high intensity discharge lamp 312 having at least one predominant peak in the 390 to 430nm range. Both the high intensity discharge lamp 304 and 312 are fixedly mounted within the apparatus 300.

The electronic module 310 is coupled to a mode selection button 316 for the person's 308 selection of one of the modes of operation of the apparatus 300. For example, the mode selection button 316 has two switch positions, one for selecting the first mode of operation for skin rejuvenation treatment and the other for selecting the second mode of operation for treatment of skin disorder.

In the first mode of operation only the high intensity discharge lamp 304 is switched on whereas in the second mode of operation only the high intensity discharge lamp 312 is switched on. Alternatively the respective intensities of the high intensity discharge lamps 304, 312 are varied depending on the selected mode of operation.

Preferably, the apparatus 300 has a shield 318 that reflects light emitted from the high intensity discharge lamp 304 and/or 312 onto the reflector 306 in order to prevent glaring of the person 308. The shield 318 can be a separate component or it can be integrated into each of the high intensity discharge lamps 304, 312. Such an antiglare screen avoids direct irradiation from the lamp (the light that reaches the face, or by accident the eye, is only reflected). This can be a metal screen in front of the lamp, or a reflective layer in the front of the outer bulb of the lamp.

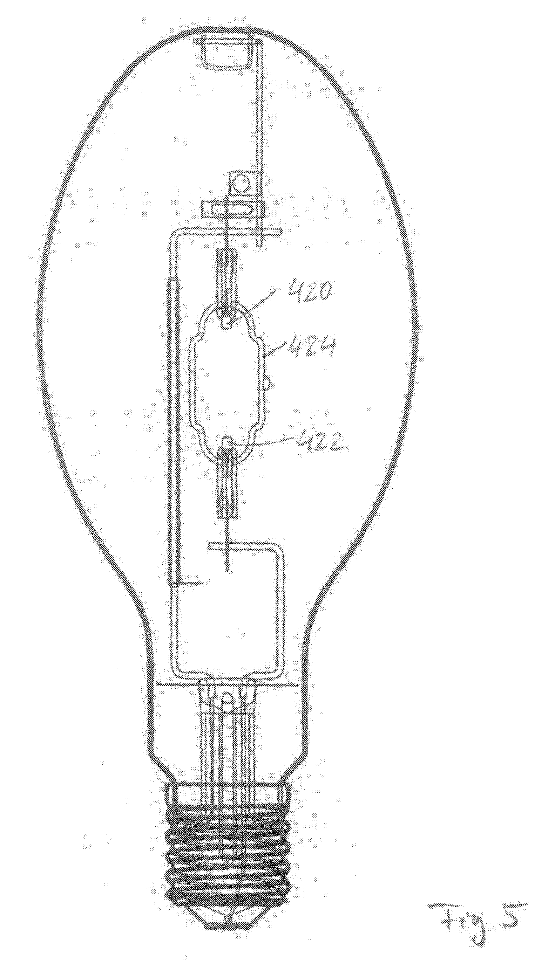

Fig. 5 shows a cross-sectional view of an embodiment of a high intensity discharge lamp. The high intensity discharge lamp has electrodes 420, 422 with a plasma chamber 424. The electrodes 420, 422 have a distance of approximately between 10 to 50 mm. The plasma chamber 424 is filled with argon, xenon, krypton and/or neon with or without mercury. In addition the plasma chamber 424 has a halide salt filling, such as lithium iodide, sodium iodide, calcium iodide, gallium iodide and/or indium iodide salt filling.

The wall of the plasma chamber 424 is made of quartz glass.

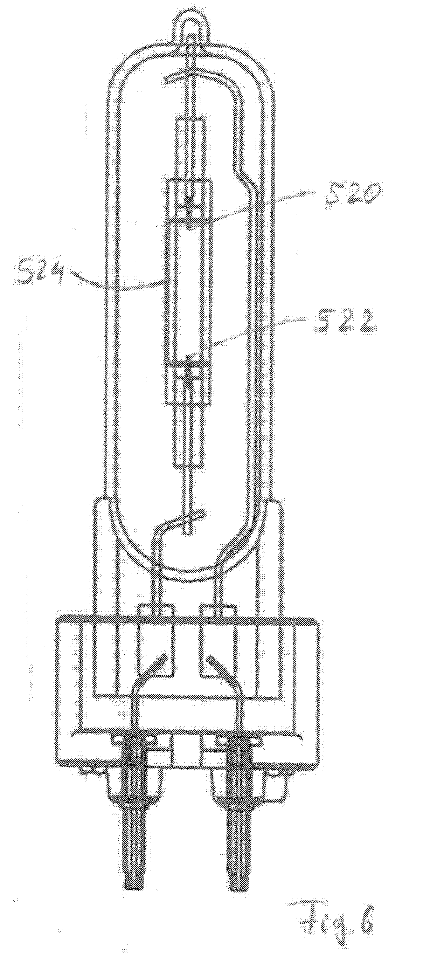

Fig. 6 shows a cross-sectional view of an alternative embodiment of a high intensity discharge lamp. In the embodiment considered here the wall of the plasma chamber 524 is made of PCA.

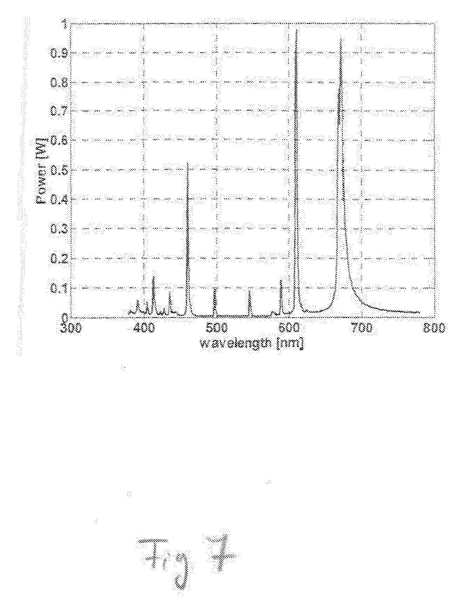

Fig. 7 shows a spectrum of a high intensity discharge lamp that is obtained with a lithium iodide salt filling. The high intensity discharge lamp has two predominant peaks in the 550 to 700nm range and can thus be used for skin rejuvenation treatment.

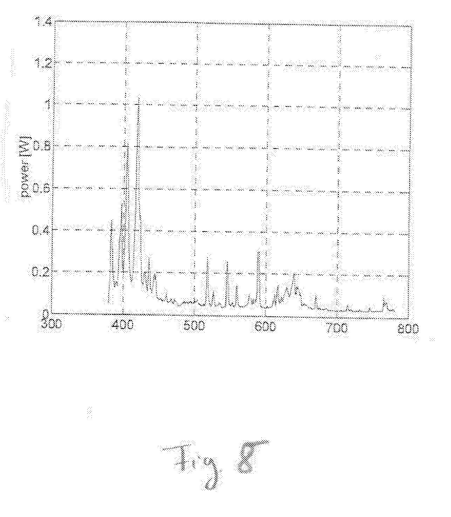

Fig. 8 shows a spectrum of a high intensity discharge lamp with a gallium iodide salt filling. The lamp has predominant peaks in the 390 to 430nm range such that it can be used for treatment of skin disorders, such as acne treatment.

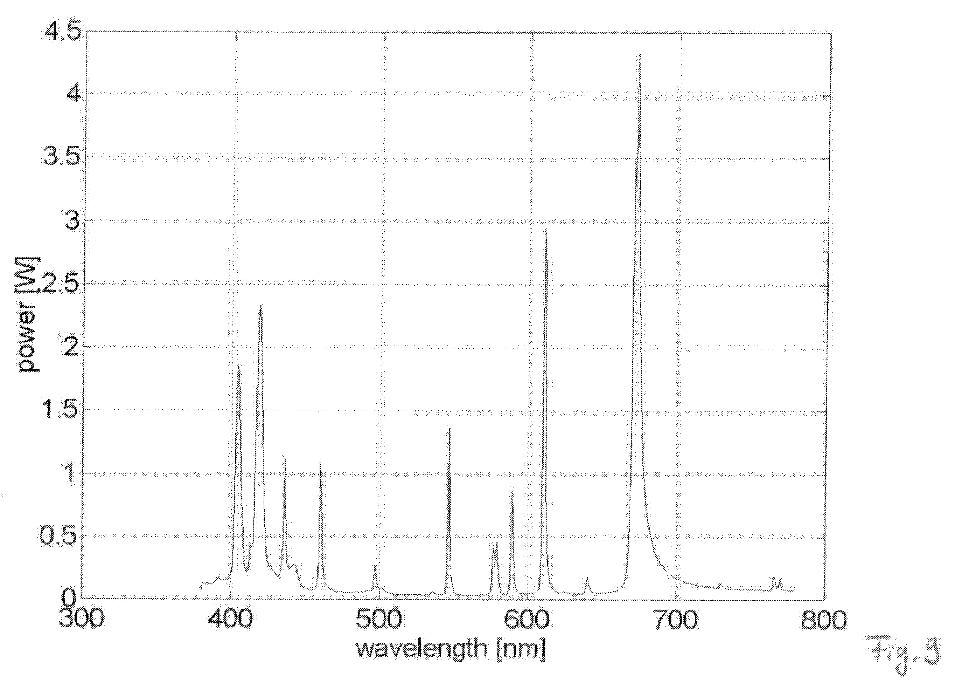

Fig. 9 shows a spectrum of a high intensity discharge lamp that has both a lithium iodide and a gallium iodide salt filling. The high intensity discharge lamp has predominant peaks in the 390 to 430nm and in the 550 to 700nm ranges and can thus very favorably be used both for treatment of skin disorders and for skin rejuvenation treatment.

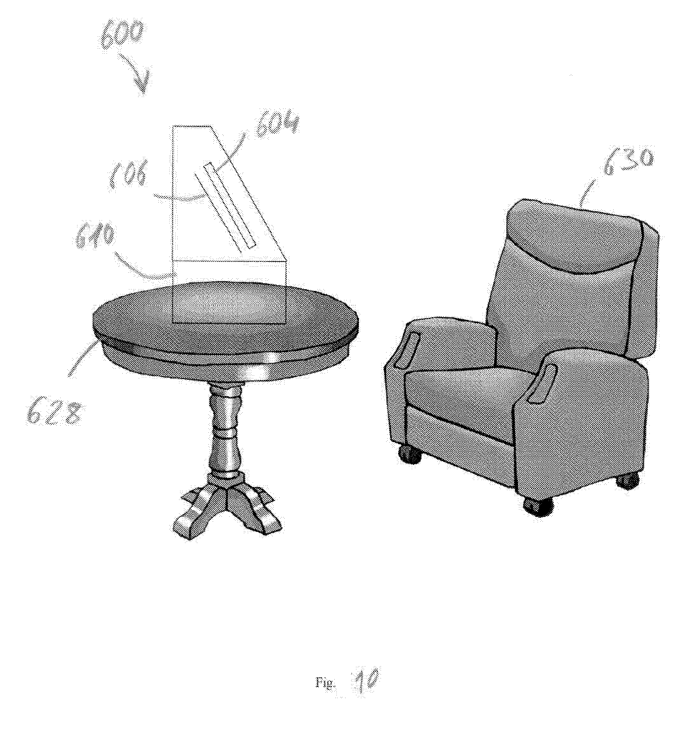

Fig. 10 shows an embodiment of apparatus 600 for home use. The apparatus 600 is portable and can be used as a table-top unit. The housing 626 of the apparatus 600 contains the electronics module 610, at least one high intensity discharge lamp 604 and a reflector 606. The reflector 606 and the at least one high intensity discharge lamp 604 are mounted in an inclined position with an inclination angle of e.g. between 10-40 degrees, preferably 30 degrees, with respect to the vertical direction. When the apparatus 600 is put on a table 628 a person can sit down on armchair 630 in order to be conveniently exposed to the radiation emitted from the apparatus 600.

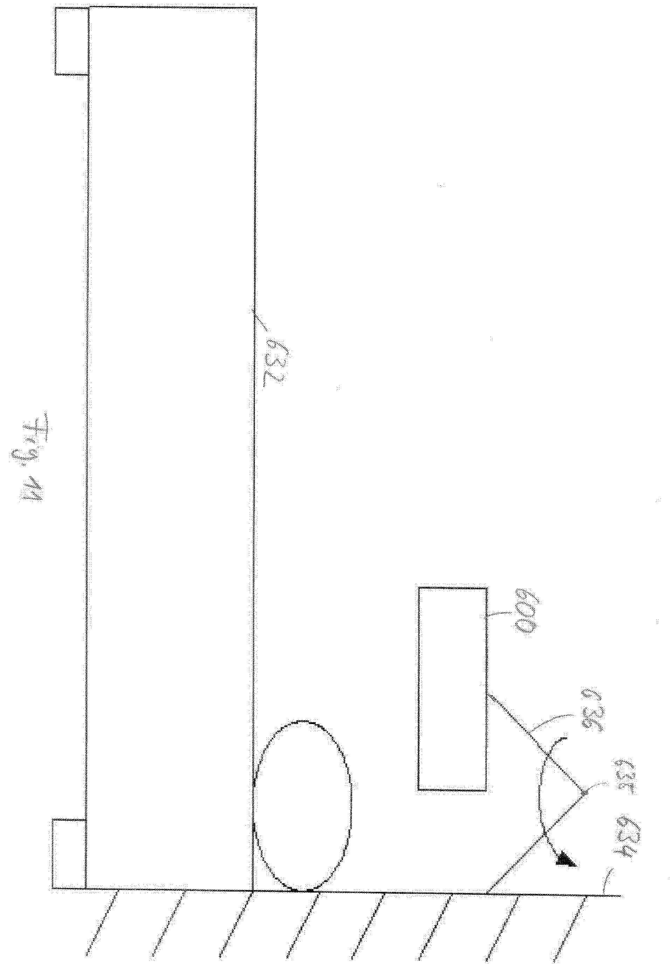

Preferably the housing of the apparatus 600 is designed to be hung to a wall 634 as shown in Fig. 11. This allows the person to lie on a bed 632 while being exposed to the radiation. In the embodiment shown in Fig. 11 the apparatus 600 has a swivelling support for fixing on wall 634 or on the ceiling. The support is pivotable about axis 638 to move apparatus 600 into a convenient use position.

It is to be noted that the apparatus 600 of the embodiments of figures 10 and 11 can be a single or dual mode apparatus, e.g. for wrinkle reduction treatment alone or for wrinkle reduction treatment and acne treatment, depending on the selected mode of operation.

### List of Reference Numerals

| | |
|---|---|
| 100 | Apparatus |
| 102 | Socket |
| 104 | High intensity discharge lamp |
| 106 | Reflector |
| 108 | Person |
| 110 | Electronics module |
| 200 | Apparatus |
| 204 | High intensity discharge lamp |
| 206 | Reflector |
| 208 | Person |
| 210 | Electronics module |
| 212 | High intensity discharge lamp |
| 214 | Axis |
| 300 | Apparatus |
| 304 | High intensity discharge lamp |
| 306 | Reflector |
| 308 | Person |
| 310 | Electronics module |
| 316 | Mode selection button |
| 318 | Shield |
| 420 | Electrode |
| 422 | Electrode |
| 424 | Plasma chamber |
| 520 | Electrode |
| 522 | Electrode |
| 524 | Plasma chamber |
| 600 | Apparatus |
| 604 | High intensity discharge lamp |
| 606 | Reflector |
| 610 | Electronics module |
| 628 | Table |
| 630 | Armchair |
| 632 | Bed |
| 634 | Wall |
| 636 | Support |
| 638 | axis |

## Claims

1. Apparatus for cosmetic skin rejuvenation treatment comprising a continuous wave light source having a high intensity discharge lamp (104; 204; 304; 604), the high intensity discharge lamp having at least one predominant spectral peak between 550 and 700nm.

2. The apparatus of claim 1, in which the high intensity discharge lamp has a salt filling, the salt filling comprising at least one component from the group consisting of lithium halide, sodium halide, calcium halide, gallium halide.

3. The apparatus of claim 1 or 2, the salt filling having at least one component from the group consisting of lithium iodide, sodium iodide, calcium iodide, gallium iodide.

4. The apparatus of claim 1, 2 or 3, the high intensity discharge lamp having a salt filing comprising lithium iodide and gallium iodide.

5. The apparatus of any one of the preceding claims, the high intensity discharge lamp having a gas filling, the gas having at least one component being selected from the group consisting of argon, xenon, krypton, neon.

6. The apparatus of any one of the preceding claims, the high intensity discharge lamp having a plasma chamber (424; 524), the plasma chamber having a quartz glass wall or a ceramic wall.

7. The apparatus of any one of the preceding claims, the continuous wave light source being adapted to operate with an alternative high intensity discharge lamp for treatment of a skin disorder.

8. The apparatus of claim 7, the alternative high intensity discharge lamp (212; 312) having a predominant spectral peak between 390 and 430nm.

9. The apparatus of claim 7 or 8, the continuous wave light source having means (102) for releasably receiving the high intensity discharge lamp or the alternative high intensity discharge lamp.

10. The apparatus of any one of the preceding claims 7 to 9, the high intensity discharge lamp having a first salt filling, such as comprising lithium iodide, and the alternative high intensity discharge lamp having a second salt filing, such as comprising gallium iodide.

11. The apparatus of any one of the preceding claims 7 to 10, the continuous wave light source having means (214) for pivoting one of the high intensity discharge lamp or the alternative high intensity discharge lamp into a use position.

12. The apparatus of any one of the preceding claims 7 to 11, the continuous wave light source comprising the high intensity discharge lamp (304) and the alternative high intensity discharge lamp (312), and a control element (316) for a user's selection of a first mode of operation or a second mode of operation, wherein in the first mode of operation at least the high intensity discharge lamp is switched on for skin rejuvenation treatment and in the second mode of operation at least the alternative high intensity discharge lamp is switched on for treatment of the skin disorder.

13. The apparatus of any one of the preceding claims being portable.

14. The apparatus of any one of the preceding claims being a table-top unit.

15. The apparatus of any one of the preceding claims being adapted to be hung to a wall or to a ceiling.

16. The apparatus of any one of the preceding claims being adapted to provide a radiation dose of at least 10 J/cm2 onto a skin area.

17. Lamp suitable for use in an apparatus according to any one of the preceding claims 2 to 16.

18. Use of an apparatus in accordance with any one of the preceding claims for anti-wrinkle treatment of a person, wherein the lamp has a salt filling comprising lithium iodide.

19. Use of an apparatus in accordance with any one of the preceding claims for acne treatment of a person.

20. Use of a high intensity discharge lamp (104; 204; 304; 604) for anti-wrinkle treatment of a person, the high intensity discharge lamp having a salt filling comprising lithium iodide.
